# EUROPEAN PATENT SPECIFICATION

(11) **EP 4 286 445 B1**
(45) Date of publication and mention of the grant of the patent: **30.09.2026**
(21) Application number: 22460052.8
(22) Date of filing: 20.10.2022
(51) Int. Cl.: C08G 61/12, C08L 65/00, C09D 165/00, H10K 30/00

(54) **NEW PERINONE POLYMER AND METHOD OBTAINING A PERINONE POLYMER IN THE PROCESS OF POTENTIOSTATIC POLYMERIZATION**
NEUES PERINON-POLYMER UND VERFAHREN ZUR HERSTELLUNG EINES PERINONPOLYMERS IM PROZESS DER POTENTIOSTATISCHEN POLYMERISATION
NOUVEAU POLYMÈRE DE PÉRINONE ET MÉTHODE D'OBTENTION D'UN POLYMÈRE PÉRINONE DANS LE PROCESSUS DE POLYMÉRISATION POTENTIOSTATIQUE

(30) Priority: 03.06.2022 PL 44137122
(43) Date of publication of application: 06.12.2023
(73) Proprietor: Politechnika Slaska, 44-100 Gliwice (PL)
(72) Inventor: LAPKOWSKI, Mieczyslaw, 44-207 Rybnik (PL); JANASIK, Patryk, Orzesze (PL); CZICHY, Malgorzata, 40-404 Katowice (PL)

(56) References cited:
- EP-A1- 0 559 392
- WO-A1-2019/057497
- DE-A1- 19 636 032
- US-A1- 2012 157 586
- CZICHY MALGORZATA ET AL: "Influence of isomeric phthaloperinone monomers on the formation of [pi]-dimers and [sigma]-bonded segments in electrochemically-crosslinked products", ELECTROCHIMICA ACTA, ELSEVIER, AMSTERDAM, NL, vol. 370, 19 January 2021 (2021-01-19), XP086497236, ISSN: 0013-4686, [retrieved on 20210119], DOI: 10.1016/J.ELECTACTA.2020.137669
- VAN DEUSEN R. L.: "Benzimidazo-benzophenanthroline polymers", vol. 4, no. 3, 1 March 1966 (1966-03-01), JP, pages 211 - 214, XP093070022, ISSN: 0449-2986, Retrieved from the Internet <URL:http://dx.doi.org/10.1002/pol.1966.110040310> DOI: 10.1002/pol.1966.110040310
- MASASHI MAMADA ET AL: "Green Synthesis of Polycyclic Benzimidazole Derivatives and Organic Semiconductors", ORGANIC LETTERS, vol. 13, no. 18, 16 September 2011 (2011-09-16), US, pages 4882 - 4885, XP055131355, ISSN: 1523-7060, DOI: 10.1021/ol201973w
- MASASHI MAMADA ET AL: "Supporting Information Green Synthesis of Polycyclic Benzimidazole Derivatives and Organic Semiconductors", 1 January 2011 (2011-01-01), XP055767088, Retrieved from the Internet <URL:https://www.supportinginformation.com> [retrieved on 20210120]
- LAPKOWSKI MIECZYSLAW: "Perinone-New Life of an Old Molecule", MATERIALS, vol. 14, no. 22, 1 January 2021 (2021-01-01), pages 1 - 49, XP093001548, DOI: 10.3390/ma14226880

## Description

The subject of the patent is a novel perinone polymer and a method of obtaining a perinone polymer in the process of potentiodynamic polymerization.

Π-conjugated polymers have wide range of applications due to their ability of electrical conduction. This property can be tailored by physical agents and / or chemical modifications (Dengke Zhaoa, Ligui Li, Wenhan Niua, Shaowei Chena, Highly conductive polythiophene films doped with chloroauric acid for dual-mode sensing of volatile organic amines and thiols, Sensors and Actuators B: Chemical 2017, 243, 380-387, doi: 10.1016/j.snb.2016.12.018). The increase in electrical conductivity could be of a mixed nature in which electron holes, electrons, ions or electron-hole pairs (so-called excitons) serve as the current carriers. In the case of semiconductors, controlling the conductive properties is crucial because it enables their specific application in electronics and optoelectronics. Doping process increases both the electronic and ionic conductivity of organic semiconductors, but only a few of polymers can be reduced, and even fewer of them demonstrate stable conductivity in the reduced state. Therefore, stable electron conductors that are effective in a neutral state and functional at room temperature are particularly challenging to obtain. The fundamental problem is to achieve a π-conjugated polymer with high electron conductivity in the neutral state. Increasing the electron conductivity of π-conjugated polymers can be attain by increasing the effective delocalization length of the main chain, side substituent effects, increasing long-range interactions, obtaining a donor-acceptor, ladder or partially ladder structure e.g. poly(benzobisimidazobenzophenanthroline) BBL (CLP - Conjugated Ladder Polymers) (R. L. Van Deusen, R., Benzimidazo-benzophenanthrolinepolymers, Journal of Polymer Science Part B: Polymer Letters 1966, 4(3), 211-214; doi: 10.1002/pol.1966.110040310; Masashi Mamada, Cesar Perez-Bolivar, Pavel Anzenbacher Jr, Green Synthesis Of Polycyclic Benzimidazole Derivatives And Organic SemiconductorsOrganic Semiconductors, Organic Letters 2011, 13(18), 4882-4885, doi: 10.1021/ol201973w). CLPs differ from conventional conductive polymers in that their backbones contain fused aromatic rings. Torsional rotation between aromatic units along the condutedconducted backbone is restricted by this structure, for example, ladder polymers based on polyquinoxaline (J. K. Stille and E. L. Mainen , J. Polym. Sci., Part B: Polym. Lett., 1966, 4 , 39 -41), and poly(phenothiazine) (M. D. Pace, O. K. Kim, (1988). (Photo)Conducting conducting properties and electron paramagnetic resonance of other ladder structures were also known earlier, e.g. pof polyphenothiazine and polyphenoxazine ladder polymersolyphenothiazine and polyphenoxazine polymers (, Synth. Met, 1988, 25, 333-339. doi:10.1016/0379-6779(88)90573-5). Processes for obtaining many bezimidazoles were already known and carried out mainly on the basis of polycondensation processes using tetraamines (EP 0 559 392 A1 (XEROX CORPORATION) Photoconductive imaging members with ladder polymers (1993.09.08); US 2012/157586 A1 (DERN/GESA [DE] EL AL] (2012.06.21), DE 196 36 032 A1 Bridged Perinone (BAYER AG[DE] ) (1998.03.12)). Another approach was to obtain monomers from diamines in the condensation process, which could then be polymerized. , poly(phenothiazine), and carbazole. Findings in the literature also reports the reactivity of perinone monomers obtained by condensation from 1,8-naphthalenediamine (Czichy, M.; Motyka, R.; Zassowski, P.; Grabiec, E.; Janasik, P.; Brzeczek-Szafran, A.; Laba, K.; Wolinska-Grabczyk, A.; Lapkowski, M. Effects of solution-phase ordering on the spectroscopic properties and electrooxidative reactivity of isomeric mixtures and isolated isomers of synthesized amidine derivatives. Dye Pigment. 2020, 178, 108309 and Czichy, M.; Janasik, P.; Motyka, R.; Zassowski, P.; Grabiec, E.; Wolinska-Grabczyk, A.; Lapkowski, M. Influence of isomeric phthaloperinone monomers on the formation of π-dimers and σ-bonded segments in electrochemically-crosslinked products. Electrochim. Acta 2021, 370, 137669). For this reason, we have proposed novel perinones with perimidine terminal groups as polymer precursors, since this one consists of both a π-donor and a π-acceptor system where, lone pairs of nitrogen atoms transfer their electron density to the naphthalene ring (Mieczyslaw Lapkowski, Perinone-New Life of an Old Molecule, Materials 2021, 14(22), 6880; doi.org/10.3390/ma14226880). Therefore, there increases the simultaneously occurrence of electrophilic and nucleophilic properties and subsequent oxidative coupling reactions. The invention relates to a perinone polymer characterized by electroactivity, bipolarity, n-conductivity and a low band gap. This polymer can be used in the production of: diodes (OLED, NIR), (bio)sensors, memory devices, substitutes for metallic conductors, anti-corrosion coatings, etc. ( R. L. Van Deusen, J. Polym. Sci., Part B: Polym. Lett., 1966, 4, 211-214).

The aim of the invention is to develop a perinone polymer and a method of its preparation, which will allow to obtain a material with a high n-type conductivity and a low band gap.The content of this invention is a novel perinone polymer,and its general formula contains units marked as u, w, v, x, y, z: 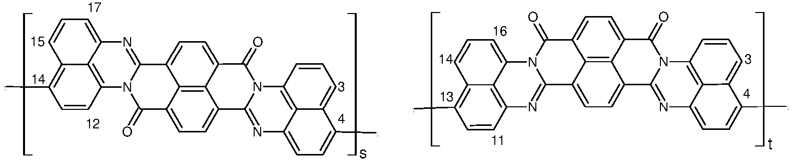 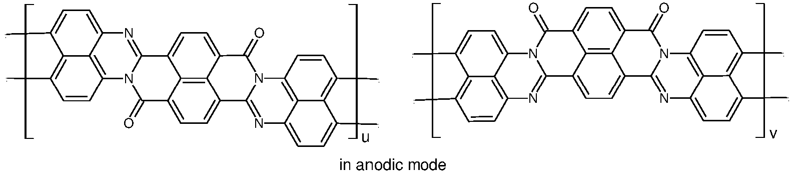 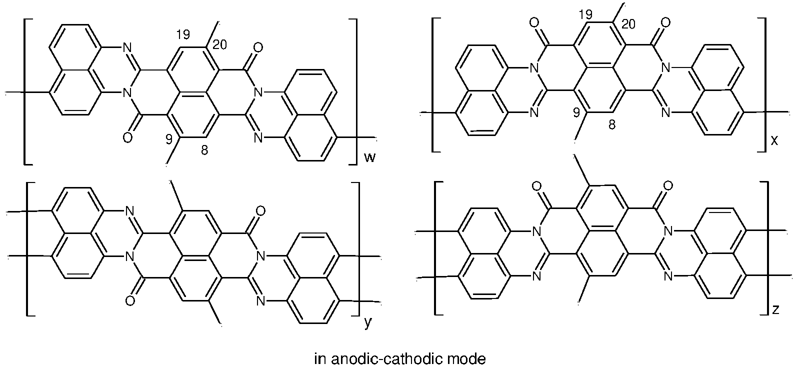 where:
s - poly(*anti*M) by single bond via positions 1, 2, 3, 4, 5, 6, 12, 13, 14, 15, 16 and/or 17
t - poly(*syn*M) by single bond via positions 1, 2, 3, 4, 5, 6, 11,12, 13, 14, 15 and/or 16
u - poly(*anti*M) by double bond via positions 1, 2, 3, 4, 5, 6, 12, 13, 14, 15, 16 and/or 17
v - poly(*syn*M) by double bond via positions 1, 2, 3, 4, 5, 6, 11,12, 13, 14, 15 and/or 16
w - poly(*anti*M) by single bond via positions 1, 2, 3, 4, 5, 6, 8, 9, 12, 13, 14, 15, 16, 17, 19 and/or 20
x- poly(*syn*M) by single bond via positions 1, 2, 3, 4, 5, 6, 8, 9, 11,12, 13, 14, 15, 16, 19 and/or 20
y - poly(*anti*M) by double bond via positions 1, 2, 3, 4, 5, 6, 8, 9, 12, 13, 14, 15, 16, 17, 19 and/or 20
z - poly(*syn*M) by double bond via positions 1, 2, 3, 4, 5, 6, 8, 9, 11, 12, 13, 14, 15, 16, 19 and/or 20.

The content of the invention is also a method of obtaining a perinone polymer in the process of potentiodynamic polymerization in the mixture of *anti*M+*syn*M (*anti* benzo[*lmn*]diperimidino[2,1-*b*:2',*1'-i*][3,8]phenanthroline-10,21-dione and *syn* benzo[*lmn*]diperimidino[2,1-*b*:1',2'-*j*][3,8]phenanthroline-18,21-dione, in a molar ratio from 1:0 to 0: 1), using cyclic voltammetry on a working electrode made of metallic or carbon materials; preferably platinum, a platinum alloy or a glassy carbon, where the process is carried out under the conditions of:
- recommended sweeping potential between 0.5 V and 1 V against the redox pair of ferrocene/ ferrocenium, reaching the oxidation peak of *anti*M+*syn*M (anodic mode),
- recommended sweeping potential between -1.75 V and 1 V against the redox pair ferrocene/ferrocenium, reaching the oxidation and reduction peak of *anti*M+*syn*M (anodic-cathodic mode),
- potential sweep, preferably in the range of 10 - 500 mV/s,
- deposition from the solution *anti*M+*syn*M with solvent as toluene or benzene and electrolyte as tetrabutylammonium tetrafluoroborate, tetrabutylammonium hexafluorophosphate or tetraammonium hexafluorophosphate,
- deposition from solid state of *anti*M+*syn*M in solvent as dichloromethane, chlorobenzene, 1,2-dichlorobenzene or methanol with electrolyte as form of tetrabutylammonium tetrafluoroborate, tetrabutylammonium hexafluorophosphate, tetraethylammonium hexafluorophosphate,
the process is carried out until the PPBF product is deposited on the working electrode.

### Example I.

The perinone polymer with a poly(perimidinobenzophenanthroline) skeleton (PPBF) was obtained by electropolymerization from a mixture of benzo[*lmn*]diperimidino[2,1-*b*:2',1'-*i*][3,8]phenanthroline-10,21-dione and benzo[*lmn*]diperimidino[2,1-*b*:1',2'-*j*][3,8]phenanthroline-18,21-dione. The PPBF polymer is electrically conductive, has complex redox activity, mixed conductivity and a low bad gap.

### Materials

The following materials were used: naphthalene-1,4,5,8-tetracarboxylic dianhydride (1); 1,8-naphthalenediamine (2); tetrabutylammonium tetrafluoroborate; dichloromethane; toluene; benzene; chlorobenzene; 1,2-dichlorobenzene; nitrobenzene; methanol and isobutanol.

### Synthesis of monomers antiM+synM

Mixture of monomers (*anti*M+*syn*M) as a mixture of *anti* isomer benzo[*lmn*]diperimidino [2,1-*b*:2',1'-*i*][3,8]phenanthroline-10,21-dione and *syn* isomer benzo[*lmn*]diperimidino[2,1-*b*:1',2'-*j*][3,8]phenanthroline-18,21-dione was obtained by condensation reaction of 1 compound (0.270 g, 1 mmol/L) with 2 compound (0.316 g, 2 mmol/L) (FIG. 1). The reaction was carried out in 10 ml of isobutanol for 24h under reflux. After the reaction was complete, the mixture was allowed to cool to room temperature. The precipitate was then filtered off and washed with methanol and then was first heated up in hexane for 1h and after in methanol for 1h under reflux. The precipitate was filtered off and dried under vacuum. *Anti*M+*syn*M was obtained as a black powder (0,399 g, 78%) Tₘ: >450 °C. ¹H-NMR: δ/ppm (300 MHz, CDCl₃ with the addition of 10%-CF₃COOD, Me₄Si, mixture of *anti* and *syn* isomers) = 1.05 (CF₃COOH); 9.28 (m, 2H); 9.05 (m, 2H); 8.59 (m, 2H); 7.76 (m, 4H); 7.65 (m, 2H); 7.56-7.48 (m, 4H). IR: vₘₐₓ/cm⁻¹ = 1678, 1624, 1595, 1548, 1528, 1492, 1469, 1394, 1369, 1342, 1319, 1284, 1257, 1225, 1165, 1121, 1106, 1072, 1043, 1003, 905, 865, 829, 793, 752.

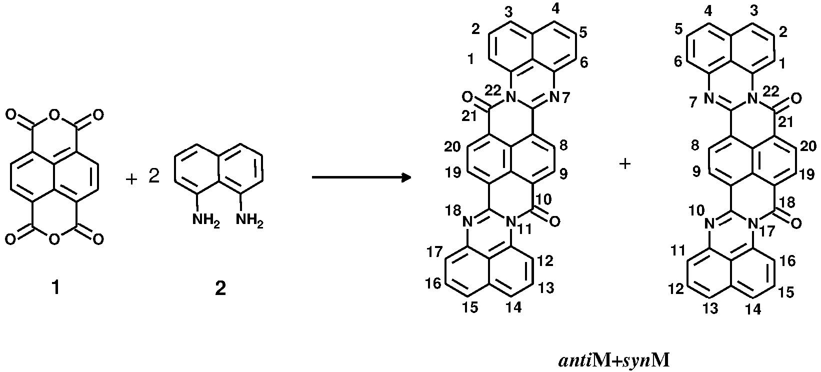

FIG.1. Diagram showing the synthesis of *anti*M+*syn*M monomer mixture for the preparation of the PPBF polymer.

### Preparation of PPBF polymer

PPBF is obtained under potentiodynamic polymerization (cyclic voltammetry) in a solution of *anti*M+*syn*M and an electrolyte, preferably tetrabutylammonium tetrafluoroborate in dichloromethane, chlorobenzene or 1,2-dichlorobenzene. Potentiodynamic synthesis of PPBF can be performed in the anodic mode (FIG. 2a) or in the anodic-cathodic mode (FIG. 2b). Obtaining of PPPI in anodic mode must provide a potential sweep with a range of potentials including the oxidation peak of *anti*M+*syn*M. Obtaining of PPBF in the anodic-cathodic mode must provide a potential sweep within the potential limits of the oxidation and the reduction peak of *anti*M+*syn*M. The oxidation peak of *anti*M+*syn*M begins above 0.45 V versus the redox pair of ferrocene/ferrocenium couple. The first reduction peak of *anti*M+*syn*M begins below -0.65 V versus redox pair of ferrocene/ferrocene couple. The PPBF polymer can be deposited at different potential sweep rates on a various metallic, carbon, organic and inorganic surfaces that are electrically conductive. The polymerization can also be carried out in the solid state using the potentiodynamic or potentiostatic method.

PPBF polymer is characterized by wide charging currents in the potential range from -2 V to 1.7 V with reversible redox pairs at 0.10/0.15 V; 0.35/0.25 V, -0.95/-1.00 and -1.35/-1.40 V V against the redox ferrocene/ferrocenium pair. The levels of the HOMO, LUMO and LUMO+1 orbitals were calculated for the *anti*M+*syn*M precursors and alternative products, and thus the poly(perimidinopyrrolo[3,4-*f*] isoindole) skeleton structure was found in the PPBF product as shown in FIG. 3.

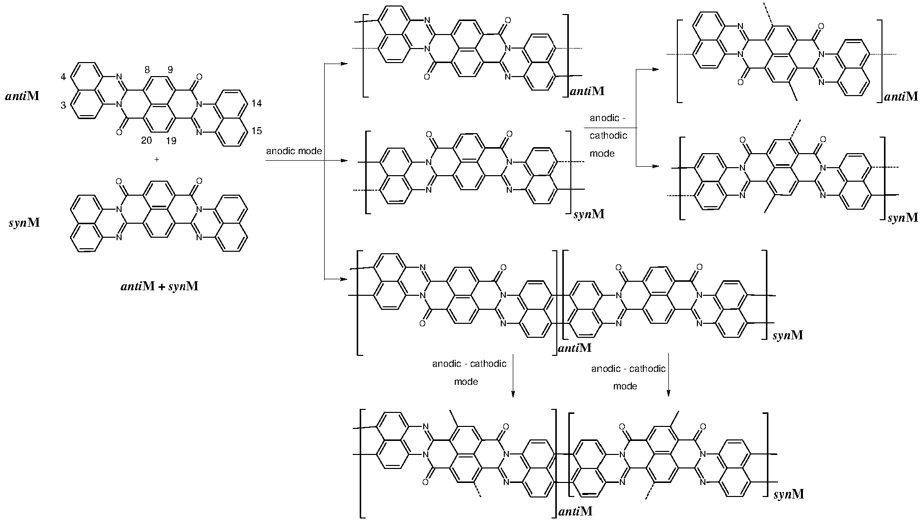

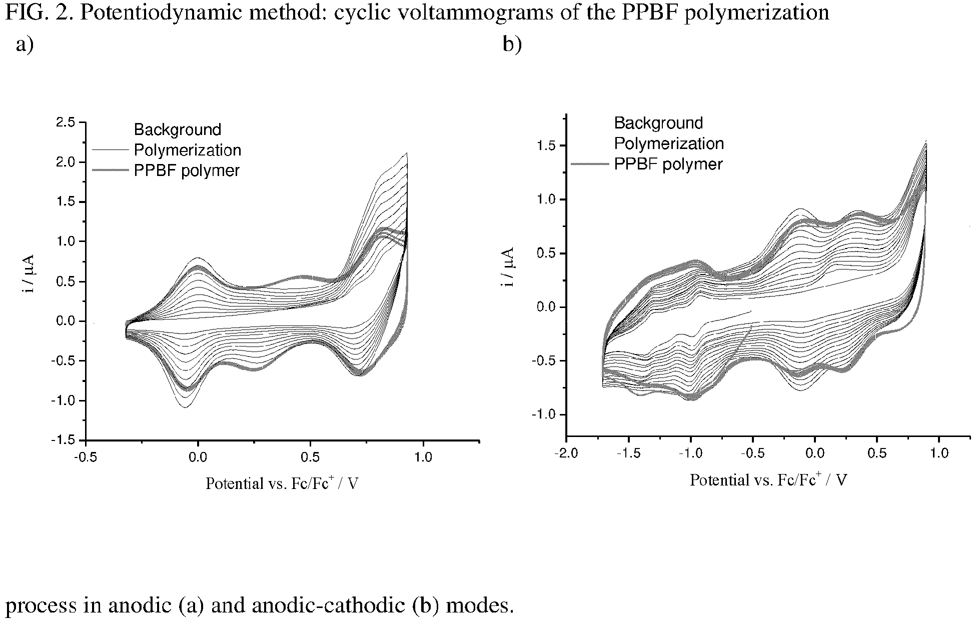

FIG. 3 Diagram of the mechanism of electrochemical preparation of PPBF and possible structure of PPBF obtained in anodic and anodic-cathodic modes.

### Method for PPBF obtaining

A mixture of *anti*M+*syn*M monomers (*anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio in the range of 1:0 to 0:1), is subjected to potentiodynamic polymerization (cyclic voltammetry) under the following conditions:
- sweeping potential between 0.5 V and 1 V against the redox pair of ferrocene/ ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential in the range of 10 - 500 mV/s,
- material of the working electrode - metallic or made of carbon materials, e.g. platinum, platinum alloys, glassy carbon etc.,
- deposition from the solution of *anti*M+*syn*M, solvents, preferably toluene, benzene, and an electrolyte that is dissolved in a solvent, preferably tetrabutylammonium tetrafluoroborate, tetrabutylammonium hexafluorophosphate or tetraethylammonium hexafluorophosphate,
- deposition from solid state of *anti*M+*syn*M, solvents, e.g. dichloromethane, chlorobenzene, 1,2-dichlorobenzene, methanol and electrolyte which is dissolved in a solvent, e.g. tetrabutylammonium tetrafluoroborate, tetrabutylammonium hexafluorophosphate, tetraethylammonium hexafluorophosphate,
- time of the process - up to the precipitation of the PPBF product on the working electrode.

As a result of the method, depending on the mode type (anodic or anodic-cathodic mode), the following perinone polymer was formed with the formula: 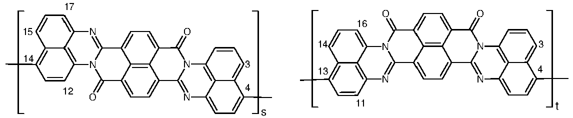 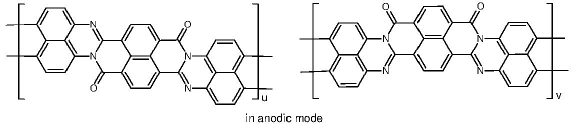 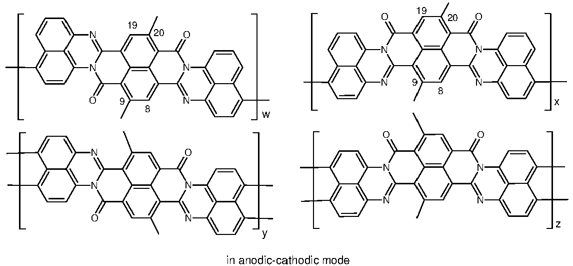 where:
s - poly(*anti*M) by single bond via positions 1, 2, 3, 4, 5, 6, 12, 13, 14, 15, 16 and/or 17
t - poly(*syn*M) by single bond via positions 1, 2, 3, 4, 5, 6, 11,12, 13, 14, 15 and/or 16
u - poly(*anti*M) by double bond via positions 1, 2, 3, 4, 5, 6, 12, 13, 14, 15, 16 and/or 17
v - poly(*syn*M) by double bond via positions 1, 2, 3, 4, 5, 6, 11,12, 13, 14, 15 and/or 16
w - poly(*anti*M) by single bond via positions 1, 2, 3, 4, 5, 6, 8, 9, 12, 13, 14, 15, 16, 17, 19 and/or 20
x - poly(*syn*M) by single bond via positions 1, 2, 3, 4, 5, 6, 8, 9, 11,12, 13, 14, 15, 16, 19 and/or 20
y - poly(*anti*M) by double bond via positions 1, 2, 3, 4, 5, 6, 8, 9, 12, 13, 14, 15, 16, 17, 19 and/or 20
z - poly(*syn*M) by double bond via positions 1, 2, 3, 4, 5, 6, 8, 9, 11, 12, 13, 14, 15, 16, 19 and/or 20

### Example II.

Example II differs from the I in that the PPBF is obtained using the following procedure.

A mixture of *anti*M+*syn*M monomers *(anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio of 1:0), is subjected to potentiodynamic polymerization (cyclic voltammetry) under the conditions:
- sweeping potential between 0.5 V and 1 V against the redox pair of ferrocene/ ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential: 10 mV/s,
- material of the working electrode - platinum,
- deposition from the solution of *anti*M+*syn*M in toluene with tetrabutylammonium tetrafluoroborate electrolyte, which is dissolved in this solvent,
- deposition from solid state of *anti*M+*syn*M, in dichloromethane and tetrabutylammonium tetrafluoroborate electrolyte, which is dissolved in this solvent,
- time of the process - up to the precipitation of the PPPI product on the working electrode.

### Example III.

A mixture of *anti*M+*syn*M monomers (*anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio of 0:1), is subjected to potentiodynamic polymerization (cyclic voltammetry) under the conditions:
- sweeping potential between 0.5 V and 1 V against the redox pair of ferrocene/ ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential: 500 mV/s,
- material of the working electrode - platinum alloys,
- deposition from the solution of *anti*M+*syn*M in benzene with tetrabutylammonium hexafluorophosphate electrolyte, which is dissolved in this solvent,
- deposition from solid state of *anti*M+*syn*M, in chlorobenzene with tetrabutylammonium hexafluorophosphate electrolyte, which is dissolved in this solvent,
- time of the process - up to the precipitation of the PPBF product on the working electrode.

### Example IV.

Example IV differs from the I in that the PPBF is obtained using the following procedure.

A mixture of *anti*M+*syn*M monomers (*anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio of 0.5:0.5), is subjected to potentiodynamic polymerization (cyclic voltammetry) under the conditions:
- sweeping potential between 0.8 V and 1 V against the redox pair of ferrocene/ ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential: 250 mV/s,
- material of the working electrode - glassy carbon,
- deposition from the solution of *anti*M+*syn*M in toluene with tetraethylammonium hexafluorophosphate electrolyte, which is dissolved in this solvent,
- deposition from solid state of *anti*M+*syn*M, in 1,2-chlorobenzene with tetraethylammonium hexafluorophosphate, tetrabutylammonium hexafluorophosphate or tetraethylammonium hexafluorophosphate electrolyte, which is dissolved in this solvent,
- time of the process -up to the precipitation of the PPBF product on the working electrode.

## Claims

1. A new perinone polymer with the general formula containing units marked as s, t, u, v, w, x, y, z: 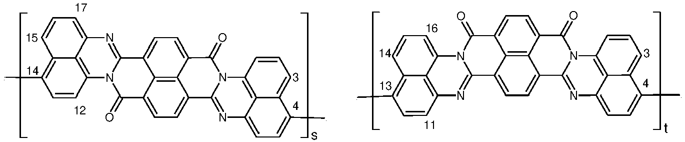 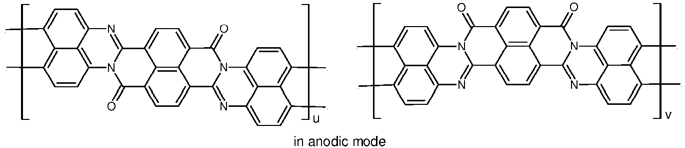 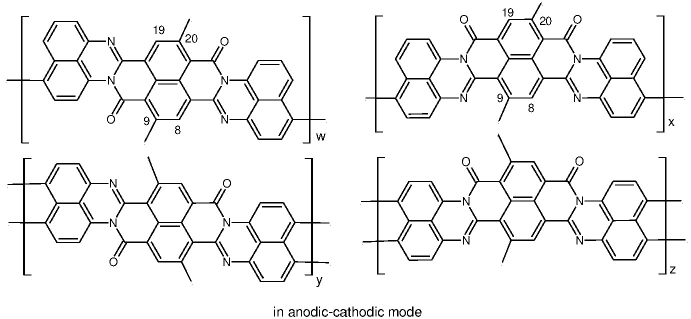 where:
s - poly(*anti*M) by single bond via positions 1, 2, 3, 4, 5, 6, 12, 13, 14, 15, 16 and/or 17
t - poly(*syn*M) by single bond via positions 1, 2, 3, 4, 5, 6, 11,12, 13, 14, 15 and/or 16
u - poly(*anti*M) by double bond via positions 1, 2, 3, 4, 5, 6, 12, 13, 14, 15, 16 and/or 17
v - poly(*syn*M) by double bond via positions 1, 2, 3, 4, 5, 6, 11,12, 13, 14, 15 and/or 16
w - poly(*anti*M) by single bond via positions 1, 2, 3, 4, 5, 6, 8, 9, 12, 13, 14, 15, 16, 17, 19 and/or 20
x- poly(*syn*M) by single bond via positions 1, 2, 3, 4, 5, 6, 8, 9, 11,12, 13, 14, 15, 16, 19 and/or 20
y - poly(*anti*M) by double bond via positions 1, 2, 3, 4, 5, 6, 8, 9, 12, 13, 14, 15, 16, 17, 19 and/or 20
z - poly(synM) by double bond via positions 1, 2, 3, 4, 5, 6, 8, 9, 11, 12, 13, 14, 15, 16, 19 and/or 20

2. Method for obtaining a perinone polymer by potentiodynamic polymerization, which is characterized as the mixture o*f anti*M+*syn*M monomers *anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio in the range 1:0 to 0:1), is subjected to potentiodynamic polymerization cyclic voltammetry on a working electrode made of metallic or carbon materials, preferably platinum, a platinum alloy or a glassy carbon, where the process is carried out under the following conditions:
- sweeping potential between 0.5 V and 1 V against the redox pair of ferrocene/ ferrocenium, reaching the oxidation peak of *anti*M+*syn*M anodic mode,
- sweeping potential between -1.75 V and 1 V against the redox pair ferrocene/ferrocenium, reaching the oxidation and reduction peak of *anti*M+*syn*M,
- speed rate of the potential in the range of 10 - 500 mV/s,
- deposition from the solution of *anti*M+*syn*M, solvents, preferably toluene, benzene, and an electrolyte that is dissolved in a solvent, preferably tetrabutylammonium tetrafluoroborate, tetrabutylammonium hexafluorophosphate or tetraethylammonium hexafluorophosphate,
- deposition from solid state of *anti*M+*syn*M, solvents, e.g. dichloromethane, chlorobenzene, 1,2-dichlorobenzene, methanol and electrolyte which is dissolved in a solvent, e.g. tetrabutylammonium tetrafluoroborate, tetrabutylammonium hexafluorophosphate, tetraethylammonium hexafluorophosphate,
the process is carried out until the PPBF product is precipitated on the working electrode.

## Patentansprüche

1. Neues Perinonpolymer mit der allgemeinen Formel, enthaltend die mit s, t, u, v, w, x, y, z bezeichneten Einheiten: 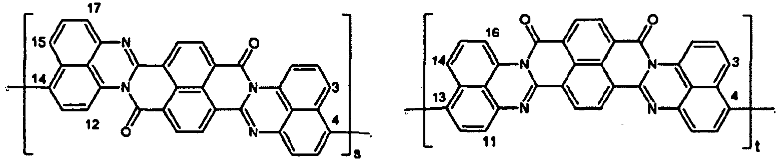 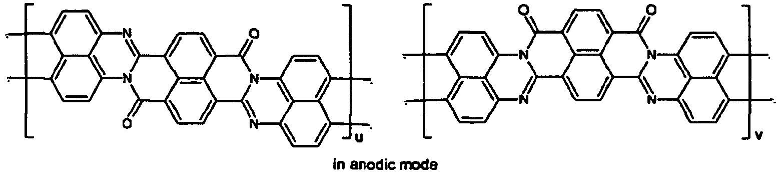 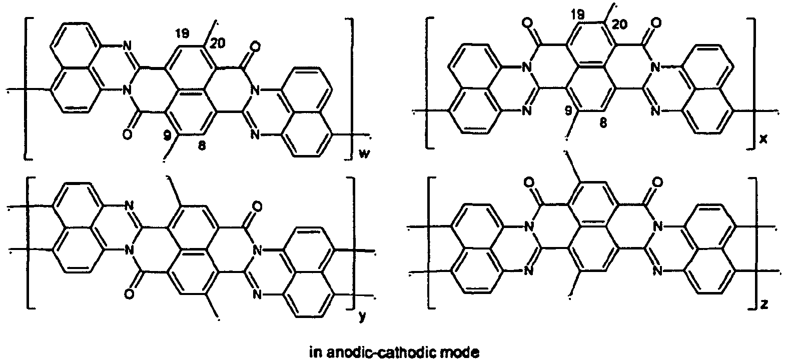 wobei:
s - poly(antiM) über Einfachbindung an den Positionen 1, 2, 3, 4, 5, 6, 12, 13, 14, 15, 16 und/oder 17
t - poly(synM) über Einfachbindung an den Positionen 1, 2, 3, 4, 5, 6, 11,12, 13, 14, 15 und/oder 16
u - poly(antiM) über Doppelbindung an den Positionen 1, 2, 3, 4, 5, 6, 12, 13, 14, 15, 16 und/oder 17
v - poly(synM) über Doppelbindung an den Positionen 1, 2, 3, 4, 5, 6, 11,12, 13, 14, 15 und/oder 16
w - poly(antiM) über Einfachbindung an den Positionen 1, 2, 3, 4, 5, 6, 8, 9, 12, 13, 14, 15, 16, 17, 19 und/oder 20
x- poly(synM) über Einfachbindung an den Positionen 1, 2, 3, 4, 5, 6, 8, 9, 11,12, 13, 14, 15, 16, 19 und/oder 20
y - poly(antiM) über Doppelbindung an den Positionen 1, 2, 3, 4, 5, 6, 8, 9, 12, 13, 14, 15, 16, 17, 19 und/oder 20
z - poly(synM) über Doppelbindung an den Positionen 1, 2, 3, 4, 5, 6, 8, 9, 11, 12, 13, 14, 15, 16, 19 und/oder 20

2. Verfahren zur Herstellung eines Perinonpolymers durch potentiodynamische Polymerisation, **dadurch gekennzeichnet, dass** ein Gemisch aus antiM+synM-Monomeren (anti-Benzo[lmn]diperimidino[2,1-b:2',1'-i][3,8]phenanthrolin-10,21-dion und syn-Benzo[lmn]diperimidino[2,1-b:1',2'-j][3,8]phenanthrolin-18,21-dion, vorzugsweise in einem molaren Verhältnis im Bereich von 1:0 bis 0:1) einer potentiodynamischen Polymerisation (zyklische Voltammetrie) an einer Arbeitselektrode aus metallischen oder Kohlenstoffmaterialien, vorzugsweise Platin, einer Platinlegierung oder Glaskohlenstoff, unterzogen wird, wobei der Prozess unter den folgenden Bedingungen durchgeführt wird:
- Durchfahren des Potentials zwischen 0,5 V und 1 V gegenüber dem Redoxpaar Ferrocen/Ferrocenium bis zum Erreichen des Oxidationspeaks von antiM+synM, anodischer Modus,
- Durchfahren des Potentials zwischen -1,75 V und 1 V gegenüber dem Redoxpaar Ferrocen/Ferrocenium bis zum Erreichen des Oxidations- und Reduktionspeaks von antiM+synM,
- Vorschubgeschwindigkeit des Potentials im Bereich von 10 - 500 mV/s,
- Abscheidung aus der Lösung von antiM+synM, Lösungsmitteln, vorzugsweise Toluol, Benzol, und einem in einem Lösungsmittel gelösten Elektrolyten, vorzugsweise Tetrabutylammoniumtetrafluoroborat, Tetrabutylammoniumhexafluorophosphat oder Tetraethylammoniumhexafluorophosphat,
- Abscheidung aus dem festen Zustand von antiM+synM, Lösungsmitteln, z. B. Dichlormethan, Chlorbenzol, 1,2-Dichlorbenzol, Methanol, und einem in einem Lösungsmittel gelösten Elektrolyten, z. B. Tetrabutylammoniumtetrafluoroborat, Tetrabutylammoniumhexafluorophosphat, Tetraethylammoniumhexafluorophosphat,
der Prozess wird durchgeführt, bis das PPBF-Produkt auf der Arbeitselektrode abgeschieden wird.

## Revendications

1. Nouveau polymère de périnone avec la formule générale contenant les unités marquées comme s, t, u, v, w, x, y, z : 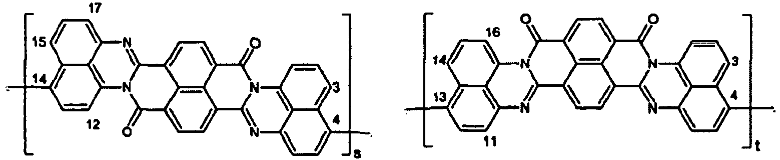 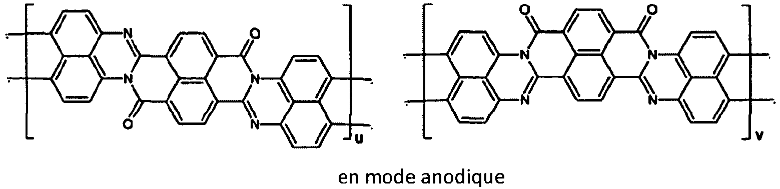 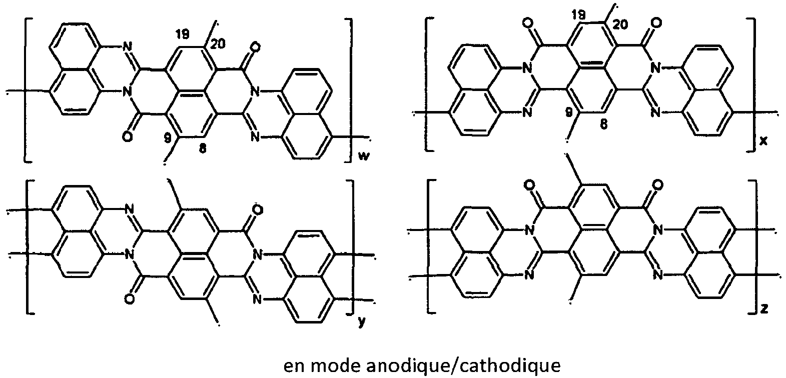 où :
s - poly(antiM) lié par liaison simple via les positions 1, 2, 3, 4, 5, 6, 12, 13, 14, 15, 16 et/ou 17
t - poly(synM) lié par liaison simple via les positions 1, 2, 3, 4, 5, 6, 11, 12, 13, 14, 15 et/ou 16
u - poly(antiM) lié par liaison double via les positions 1, 2, 3, 4, 5, 6, 12, 13, 14, 15, 16 et/ou 17
v - poly(synM) lié par liaison double via les positions 1, 2, 3, 4, 5, 6, 11, 12, 13, 14, 15 et/ou 16
w - poly(antiM) lié par liaison simple via les positions 1, 2, 3, 4, 5, 6, 8, 9, 12, 13, 14, 15, 16, 17, 19 et/ou 20
x - poly(synM) lié par liaison simple via les positions 1, 2, 3, 4, 5, 6, 8, 9, 11, 12, 13, 14, 15, 16, 19 et/ou 20
y - poly(antiM) lié par liaison double via les positions 1, 2, 3, 4, 5, 6, 8, 9, 12, 13, 14, 15, 16, 17, 19 et/ou 20
z - poly(synM) lié par liaison double via les positions 1, 2, 3, 4, 5, 6, 8, 9, 11, 12, 13, 14, 15, 16, 19 et/ou 20.

2. Méthode d'obtention de polymère de périnone par la méthode de polymérisation potentiodynamique, **caractérisée en ce que** le mélange des monomères antiM + synM (anti-benzo[lmn]diperimidino[2,1-b:2',1'-i][3,8]phenanthroline-10,21-dione et syn-benzo[lmn]diperimidino[2,1-b:1',2'-j][3,8]phenanthroline-18,21-dione, de préférence dans un rapport molaire entre 1:0 et 0:1) est soumis à la polarisation potentiodynamique (voltampérométrie cyclique) à l'électrode de travail constituée en matériaux métalliques ou carbonés, de préférence en platine, en alliage de platine ou en carbone vitreux, ledit processus est effectué dans les conditions suivantes :
- balayage en potentiel dans la plage entre 0,5 V et 1 V par rapport au couple ferrocène/ferrocénium avec le pic anodique d'oxydation des monomères antiM+synM (mode anodique),
- balayage en potentiel dans la plage entre -1,75 V et 1 V par rapport au couple ferrocène/ferrocénium avec les pics d'oxydation et de réduction des monomères antiM+synM (mode anodique/cathodique),
- vitesse de variation de potentiel entre 10 et 500 mV/s,
- dépôt en solution contenant antiM+synM, le solvant, avantageusement toluène, benzène et électrolyte dilué dans le solvant, avantageusement tétrafluoroborate de tétrabutylammonium, hexafluorophosphate de tétrabutylammonium ou hexafluorophosphate de tétraéthylammonium,
- réaction à l'état solide de antiM+synM, avec application des solvants, par ex. dichlorométhane, chlorobenzène, 1,2-dichlorobenzène, méthanol et électrolyte dilué dans le solvant, p. ex. tétrafluoroborate de tétrabutylammonium, hexafluorophosphate de tétrabutylammonium, hexafluorophosphate de tétraéthylammonium,
ledit processus est effectué jusqu'à la précipitation du produit PPBF à l'électrode de travail.
